# EUROPEAN PATENT APPLICATION

(11) **EP 3 192 498 A1**
(43) Date of publication of application: **19.07.2017**
(21) Application number: 17150859.1
(22) Date of filing: 10.01.2017
(51) Int. Cl.: A61K 8/66, A61K 8/64, A61Q 11/00, A61K 38/44, A61K 38/46, A61K 38/47, A61K 38/54, A61P 1/02

(54) **ORAL CARE COMPOSITIONS**

(30) Priority: 12.01.2016 EP 16150964; 27.06.2016 EP 16176434; 27.06.2016 EP 16176452; 30.06.2016 EP 16177370
(71) Applicant: Unilever PLC, London, Greater London EC4Y 0DY (GB); Unilever N.V., 3013 AL Rotterdam (NL)
(72) Inventor: ADAMS, Suzanne, Elizabeth, Bebington, Wirral, Merseyside CH63 3JW (GB); ARNOLD, David, Stanley, Bebington, Wirral, Merseyside CH63 3JW (GB); BRADING, Melanie, Gayle, Bebington, Wirral, Merseyside CH63 3JW (GB); GREEN, Alison, Katherine, Bebington, Wirral, Merseyside CH63 3JW (GB); MURPHY, Barry, Bebington, Wirral, Merseyside CH63 3JW (GB)
(74) Representative: Tansley, Sally Elizabeth

(57) **Abstract**

A composition comprising enzymes for use in a method to increase the health related bacterial flora of the mouth.

## Description

### Field of the Invention

The present invention is concerned with oral care compositions. More particularly, the present invention is concerned with oral care compositions containing enzymes.

### Background of the Invention

Conventional thinking with respect to toothpastes is that anti-bacterials are needed to destroy the bacteria within the mouth. The present application has found that it is beneficial to adjust the balance away from bacteria associated with poor oral health in favour of those associated with good oral health. There is therefore a need for a composition that increases the level of health associated-bacteria in the mouth.

The present application relates to compositions that increase the prominence of beneficial bacterial flora in the mouth.

### Description of the Invention

The present invention relates to a composition comprising enzymes for use in a method to increase the beneficial bacterial flora of the mouth.

### Detailed Description of the Invention

The oral microbiome is highly complex and diverse community with over 700 different species having been identified. In general, health-related bacteria are facultative anaerobic or aerobic bacteria (e.g. genus *Neisseria*) and bacteria related to gum disease are anaerobic bacteria (e.g genus *Treponema*). There is therefore a need for the promotion these beneficial bacteria in the mouth.

The present application has found that compositions comprising enzymes can be used to increase the beneficial bacterial of the mouth. Preferably the beneficial bacteria are of the genus *Neisseria* and *Prevotella*, more preferably they are of the genus A composition according to claim 1 in which the bacteria flora are of the species *Neisseria flava, Neisseria (HOT499*|*sp._str._SMC_A9199), Neisseria sp._Oral_Taxon_020* and *Neisseria* sp._str._SMC_A9199, *Prevotella melaninogenica*. Other preferred beneficial bacterial species include *Streptococcus* (*mitis*|*mitis*_bv_2|*oralis*|*pneumoniae*|HOT058|HOT064|HOT070|sp._str._C300), *Lactobacillus gasseri Streptococcu*(*infantis*|*mitis*|*mitis_*bv_2|*pneumoniae*|HOT064).

Preferably the enzymes are selected from the following groups:
i) an glycoside hydrolase, more preferably an amylase, most preferably amylglucosidase;
ii) an oxoreductase acting on OH groups of donors, more preferably an oxidase with oxygen as acceptor, most preferably glucose oxidase;
iii) a hemeperoxidase more preferably a haloperoxidase most preferably a lactoperoxidase.
iv) It is particularly preferred if the enzyme is a mixture of 2 of the above, more preferably 3 of the above, most preferably a mixture of amylglucosidase, glucose oxidase and lactoperoxidase.

Preferably the total level of enzyme is from 0.01 wt% to 2wt% of the total composition. More preferably from 0.1 wt% to 1 wt% of the total composition.

Preferably the total level of glycoside hydrolase in the composition is from 0.05 to 1 wt% of the total composition.

Preferably the total level of oxoreductase acting on OG groups of donors is from 0.05 to 1 wt% of the total composition.

Preferably the total level of hemeperoxidase is from 0.0005 to 0.01 wt% of the total composition.

Preferably the weight ratio of glycoside hydrolase to other enzymes in the composition is greater than 1:1, more preferably greater than 3:2.

Compositions of the invention may also comprise other proteins such as lysozymes, lactoferrins and colostrum. Preferably these other proteins are present at total levels from 0.001 to 0.5 wt% of the total composition, preferably from 0.005 to 0.1 wt% of the total composition.

Furthermore, the oral care composition will usually contain a surfactant in an amount of from 0.2 to 5% by weight based on the total weight of the dentifrice. Preferred are nonionic surfactants such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Particularly preferred are polyethylene glycol ethers of fatty alcohols in particular polyethylene glycol ethers having from 20 to 40 ethylene oxide groups per unit. Examples of such suitable surfactant include the group of surfactants known as Steareth surfactants such as Steareth 30. Preferably the level of non-ionic surfactant is from 0.5 to 7 wt% of the total composition, more preferably from 1 to 5 wt% of the total composition.

Although other surfactants may be present it is preferred if they are limited to levels below 0.5 wt% of the total composition preferably less than 0.1 wt% of the total composition.

The oral care composition, especially in the cases of dentifrices, may also contain structurants and agents such as binders and thickening agents. These structurants will generally be present in an amount of from 0.1 to 1% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth). Natural gum based thickeners, in particular carrageenan are particularly preferred.

Examples of suitable product forms for compositions of the invention include dentifrices, mouthwashes, mouthsprays and liquid formulations.

Preferred product forms for compositions of the invention are those which are suitable for brushing and/or rinsing the surfaces of the oral cavity. Dentifrices/toothpastes are preferred.

In such preferred product forms, the amount of water and/or polyhydric alcohol will generally be at least 10 percent, preferably at least 30 percent, more preferably at least 50 percent by total weight water and/or polyhydric alcohol based on the total weight of the composition. Preferably the level of water and/or polyhydric alcohol will be less than 90 wt% of the total composition, more preferably less than 85 wt%.

An example of a preferred type of product form in the context of the present invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

A dentifrice composition according to the invention will usually contain, as the aqueous continuous phase, a mixture of water and polyhydric alcohol in various relative amounts, with the amount of water generally ranging from 10 to 50% by weight (based on the total weight of the dentifrice) and the amount of polyhydric alcohol generally ranging from 20 to 60% by weight (based on the total weight of the dentifrice).

Typical polyhydric alcohols for use in the oral care composition, particularly a dentifrice composition according to the invention include humectants such as glycerol, sorbitol, polyethylene glycol, polypropylene glycol, propylene glycol, xylitol (and other edible polyhydric alcohols), hydrogenated partially hydrolysed polysaccharides and mixtures thereof. Glycerol and sorbitol are preferred, sorbitol being particularly preferred.

The oral care composition, in particular dentifrice compositions comprises abrasive materials. Abrasive materials will generally be present in an amount of from 0.5 to 75%, preferably 3 to 60% by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof. Silicas are particularly preferred.

Compositions of the invention may also comprise thickeners such as finely divided silicas, hectorites, calcium carbonate, colloidal magnesium aluminium silicates and mixtures thereof.

Compositions of the invention may comprise a zinc ion. Preferably the sources of zinc ions are zinc chloride, zinc acetate, zinc gluconate, zinc sulphate, zinc fluoride, zinc citrate, zinc lactate, zinc oxide, zinc monoglycerolate, zinc tartrate, zinc pyrophosphate zinc maleate and mixtures thereof.

Compositions of the present invention may also contain further optional ingredients customary in the art such as fluoride ion sources, anticalculus agents, buffers, flavouring agents, sweetening agents, colouring agents, opacifying agents, preservatives, antisensitivity agents, bleaching agents, and antimicrobial agents.

To clean the surfaces of the oral cavity, the composition according to the invention is topically applied to the oral cavity surfaces to be cleaned.

Preferably the composition according to the invention is topically applied to the oral cavity surfaces to be cleaned and then agitated by brushing and/or rinsing.

The invention will now be illustrated by the following non-limiting Examples:

### Examples

A double-blind, randomised, parallel study, was conducted to collect plaque samples. Subjects brushed twice daily for 14 weeks. Samples of plaque were collected at baseline and at the end of the 14-week brushing period for microbiomics analysis. The data generated from 102 subjects, 52 using Example 1 and 50 using Example A-standard silica SLS toothpaste, completed the microbiomics analysis.

### Example 1

| **Material** | **Amount % W/W** |
|---|---|
| Sorbitol | 28.5 |
| Zinc Gluconate | 0.25 |
| Sodium Fluoride | 0.32 |
| Citric Acid | 0.25 |
| Disodium Phosphate | 0.5 |
| Titanium Dioxide | 0.5 |
| Abrasive silica | 16.0 |
| Thickening silica | 4.3 |
| Carrageenan | 0.7 |
| Glycerin | 5.0 |
| Xanthuan m gum | 1.0 |
| Steareth 30 | 3.0 |
| Amylglucosidase | 0.3 |
| Lactoperoxidase | 0.002 |
| Lysozyme (22%)*** | 0.05 |
| Glucose Oxidase | 0.14 |
| Lactoferrinss | 0.01 |
| Colostrum | 0.01 |
| Water and minors | to100.0 |

### Example A

| **Material** | **Amount (%W/W)** |
|---|---|
| Sorbitol | 45 |
| PEG-32 | 5 |
| Monosodium Phosphate | 0.1 |
| Sodium Fluoride | 0.32 |
| Titanium Dioxide | 1 |
| Thickening silica | 7.5 |
| Abrasive silica | 10 |
| Cellulose gum | 0.63 |
| Sodium Lauryl Sulphate | 1.5 |
| Water and minors | To 100 |

### Microbiomics analysis:

Plaque samples were processed in pairs (baseline and 14 weeks) with the first step being DNA extraction. This involved an overnight lysis step followed by bead beating and
purification using an automated DNA extraction robot. The resulting DNA was quantified and normalised prior to first round polymerase chain reaction (PCR) amplification performed targeting the V4-V6 region of the 16SrRNA gene. The amplicons were processed through a second round of PCR to incorporate Illumina sequencing adapter sequences containing indexes (i5 and i7) for sample identification. The resulting amplicons were then sequenced on an Illumina MiSeq with 2x300bp paired-end sequencing using v3 chemistry. Following sequencing, all the raw reads were processed simultaneously through a bioinformatics pipeline and classified to the species level against the HOMD database. Statistical analysis was performed on the resulting data sets.

### Results:

Following statistical analysis by comparative testing using Dirichlet Multinomial distribution the following changes were identified:

| **Significant Taxa** | **% Change** | **Association** |
|---|---|---|
| ***Neisseria flava*** | 2.869 | Genus associated with health |
| ***Prevotella melaninogenica*** | 0.393 | Health |
| ***Neisseria* (HOT499\|sp._str._SMC_A9199)** | 0.076 | Genus associated with health |
| ***Streptococcus* (*mitis*\|*mitis_*bv*_*2\|*oralis*\|*pneumoniae*\| HOT058\|HOT064\|HOT070\|sp._str._C300)** | 0.097 | Health |
| ***Prevotella* (*melaninogenica*\|HOT314)** | 0.036 | Health |
| ***Neisseria* sp._Oral_Taxon_020** | 0.088 | Genus associated with health |
| ***Neisseria* sp._str._SMC_A9199** | 0.001 | Genus associated with health |
| ***Streptococcus* (*infantis*\|*mitis*\|*mitis_*bv*_*2\| *pneumoniae*\|HOT064)** | 0.001 | Health |
| ***Prevotella* (*histicola*\|*melaninogenica*)** | 0.003 | Health |
| ***Lactobacillus gasseri*** | 0.002 | Health |

Detailed analysis of the species overtime showed a significant increase in health related bacteria.

## Claims

1. A composition comprising enzymes for use in a method to increase the health related bacterial flora of the mouth.

2. A composition according to claim 1 in which the bacterial flora are of the genus *Neisseria* and *Prevotella.*

3. A composition according to claim 1 in which the bacteria flora are of the species *Neisseria flava, Neisseria (HOT499*|*sp._str._SMC_A9199), Neisseria sp._Oral_Taxon_020* and *Neisseria* sp._str._SMC_A9199, *Prevotella melaninogenica*.

4. A composition according to any preceding claim in which the enzymes are selected from a glycoside hydrolase, an oxoreductase acting on OH groups of donors, a hemeperoxidase or mixtures thereof.

5. A composition according to claims 1 to 3 in which the enzymes are selected from an amylase, an oxidase with oxygen as acceptor, a haloperoxidase or mixtures thereof.

6. A composition according to claims 1 to 3 n which the enzyme is selected from the group consisting of amylglucosidase, glucose oxidase, lactoperoxidase or mixtures thereof.

7. A composition according to any preceding claim in which the composition comprises a mixture of two enzymes described in any previous claims.

8. A composition according to any preceding claim in which the composition comprises a mixture of three enzymes described in any preceding claim.

9. A composition according to any preceding claim which comprises a zinc ion.

10. A composition according to any preceding claim in which the composition further comprises a non-ionic surfactant.

11. A composition according to claim 10 in which the non-ionic surfactant is a polyethylene glycol ethers of a fatty alcohol.

12. A composition according to claim 10 in which the non-ionic surfactant is Steareth 30.

13. A composition according to any preceding claim in which the composition further comprises a further protein.

14. A composition according to claim 13 in which the protein comprise glycoside hydrolase.

15. A composition according to any preceding claim in which the composition comprises a carrageenan based thickener.

16. A composition according to any preceding claim which is in the form of a dentifrice.
